Europäisches Patentamt

European Patent Office

Office européen des brevets

(11) Numéro de publication: **0 408 416 A1**

# DEMANDE DE BREVET EUROPEEN

(21) Numéro de dépôt: 90401875.1

(22) Date de dépôt: 28.06.90

(51) Int. Cl.⁵: **A61B 17/16**

(30) Priorité: 11.07.89 FR 8909325

(43) Date de publication de la demande:
16.01.91 Bulletin 91/03

(84) Etats contractants désignés:
**BE CH DE ES FR GB IT LI SE**

(71) Demandeur: **Laboureau, Jacques-Philippe**
**24 rue de la Fontaine Billenois**
**F-21000 Dijon(FR)**

(72) Inventeur: **Laboureau, Jacques-Philippe**
**24 Rue Fontaine Billenois**
**F-21000 Dijon(FR)**
Inventeur: **Cazenave, Alain**
**22bis Rue du Docteur Calmette, Résidence**
**Suisse**
**F-21000 Dijon(FR)**

(74) Mandataire: **Bruder, Michel et al**
**Cabinet Michel Bruder Conseil en Brevets**
**10, rue de la Pépinière**
**F-75008 Paris(FR)**

(54) Instrument ancillaire chirurgical pour le repérage et le forage des tunnels d'insertion fémorale et tibiale d'un néo-ligament du genou.

(57) Instrument ancillaire chirurgical pour le repérage et le forage des tunnels d'insertion fémorale et tibiale d'au moins un faisceau de néo-ligaments pour la reconstruction du ligament croisé antérieur du genou, caractérisé en ce que ledit instrument comprend une poignée (5, 14) munie à l'une de ses extrémités d'une tige rectiligne (3, 12) terminée en crochet (4, 13) perpendiculaire à ladite tige servant de référence pour au moins un canon de visée (8, 17, 23) prenant par rapport à ladite tige (3, 12) des directions horizontales et verticales pré-déterminées de telle manière à obtenir un alignement des directions de forage des tunnels d'insertion fémorale et tibiale lorsque le genou est en extension.

Fig 1

La présente invention concerne un instrument ancillaire chirurgical permettant le repérage de l'orifice d'entrée du tunnel d'insertion fémoral d'un néo-ligament destiné à la reconstruction du croisé antérieur du genou, dans le respect total de son isométrie, ainsi que le guidage d'un outil pour le forage du même tunnel et permettant avantageusement le guidage d'un outil pour le forage du tunnel d'insertion tibiale du même néo-ligament.

Le problème majeur que l'on rencontre lors de la reconstruction du ligament croisé antérieur du genou est le respect de son isométrie. On entend par isométrie, le fait que la distance entre le point d'insertion du ligament dans le tibia et le point d'insertion du même ligament dans le fémur, ne doit pas varier quel que soit le degré de flexion ou d'extension du genou. On conçoit en effet que si cette isométrie n'était pas respectée, il en résulterait un manque de stabilité dans les positions où le ligament serait relâché et, au contraire, une limitation de la mobilité si, dans une certaine position, le ligament était soumis à une trop forte tension.

Généralement, l'insertion tibiale du ligament est assez facilement retrouvée, compte tenu de sa position très antérieure sur le plateau tibial. Il n'en est pas de même de l'insertion fémorale qui se fait sur la face axiale du condyle externe du fémur, dans une zone située profondément, difficilement accessible à la vue directe du chirurgien. Cette zone d'insertion fémorale est en outre très variable d'un individu à l'autre, et le choix de ce point est habituellement laissé à l'appréciation du chirurgien. Par ailleurs, il est essentiel de prévoir, lors de l'implantation d'un ligament prothétique du genou, un alignement parfait des axes longitudinaux des tunnels d'insertion tibiale et fémorale de telle sorte que, le genou étant en position d'extension, le ligament prothétique soit rectiligne, afin d'éviter toutes sollicitations inutiles dues aux berges saillantes des tunnels d'insertion.

Le premier but de la présente invention est donc de proposer la réalisation d'un instrument chirurgical permettant de repérer avec précision, selon des critères mesurables et reproductibles, la position de l'orifice d'entrée du tunnel d'insertion fémorale au niveau du condyle externe.

A cet égard, on connaît de nombreux guides de perçages, mais ces guides permettent de forer des tunnels d'insertion entre deux points arbitrairement choisis selon l'expérience du chirurgien. Aucun de ces guides ne permet actuellement de déterminer avec précision le point isométrique, qui constitue pourtant le point d'entrée obligatoire du néo-ligament, si on veut lui conserver une parfaite isométrie. Des travaux anatomiques, menés entre autres par les Inventeurs, ont permis d'établir avec certitude que la partie toute postérieure du condyle fémoral externe correspond en fait à un cadran de cercle parfait, dont le rayon est variable selon les individus. Ces mêmes travaux ont en outre permis de déterminer que le centre géométrique de ce cadran de cercle correspondait précisément au point isométrique recherché. La situation de ce point isométrique peut donc être déterminé par rapport à sa distance au rebord postérieur du condyle, dans la mesure où celle-ci peut être mesurée.

Dès lors, il suffit de pouvoir, préalablement à l'opération, déterminer pratiquement le centre du cercle dont il a été question ci-dessus, notamment en mesurant sa distance par rapport au rebord postérieur du condyle ; or, on sait parfaitement effectuer une telle mesure à partir d'une radiographie sans agrandissement, en utilisant par exemple la téléradiographie ; il suffit alors de superposer des calques transparents pour mesurer directement le rayon du cercle correspondant à la partie postérieure du condyle fémoral et en déduire son centre. A partir de ce même centre, il est aisé de tracer une ligne parallèlement au fond de l'échancrure inter-condylienne en se repérant, par exemple, à la ligne de Blumensatt, qui constitue un repère radiologique connu ; cette ligne correspond exactement à l'axe idéal pour le tunnel d'insertion fémoral, dans la reconstruction du ligament croisé antérieur du genou. Il suffit enfin de reporter la distance ainsi mesurée sur un instrument approprié muni d'un viseur, prenant référence sur le rebord postérieur dudit condyle externe pour obtenir, avec une excellente précision, lors de l'intervention chirurgicale, le point isométrique d'insertion fémorale du néo-ligament.

L'un des objets de la présente invention est donc de proposer un instrument ancillaire chirurgical d'une part pour le repérage du tunnel d'insertion fémorale d'un néo-ligament, destiné à la reconstruction du ligament croisé antérieur du genou, l'entrée inter-condylienne dudit tunnel étant située au point isométrique coïncidant avec le centre du cadran de cercle correspondant au rebord postérieur du condyle fémoral, et d'autre part pour le forage dudit tunnel selon une direction telle qu'en position d'extension du genou, le prolongement du tunnel soit sensiblement dans l'axe du tunnel d'insertion tibial, ledit instrument comprenant une tige, comportant, à son extrémité proximale, une poignée et, à son extrémité distale, un crochet recourbé suivant un angle droit, venant au travers de l'échancrure inter-condylienne, en appui sur le rebord postérieur du condyle fémoral concerné, pour procurer une référence de positionnement à un canon de visée et de guidage lié à ladite tige, instrument caractérisé en ce que ledit canon préorienté suivant des directions fixes horizontales et verticales, peut être déplacé parallèlement à la tige sur une section graduée pour être immobilisé par des moyens adéquats de telle sorte que la distance entre la face d'appui condylienne du crochet et le point d'intersection entre la direction de forage et ladite tige soit égale au rayon du cadran de cercle dont le centre est le point isométrique déterminé préalablement, par exemple, par téléradiographie.

Il ressort bien de la proposition qui vient d'être donnée, relative à une instrumentation ancillaire, que le chirurgien peut déterminer avec précision, la position du point isométrique d'insertion fémorale du néo-ligament. Lors d'une intervention chirurgicale pour laquelle le chirurgien, par exemple, a choisi la voie endo-articulaire, le genou à opérer étant dans une position telle que l'axe fémoral est sensiblement à 90° par rapport à l'axe tibial, l'opérateur introduit d'abord la tige de l'instrument suivant l'invention, dans l'échancrure inter-condylienne jusqu'à ce que la partie distale de ladite tige, recourbée suivant un angle droit, vienne, à la manière d'un crochet, s'appuyer sur le rebord postérieur d'un condyle externe du fémur. Lorsque cette partie recourbée repose correctement sur la partie postérieure dudit condyle, l'opérateur est alors en position telle que l'axe de la tige supportant ladite partie recourbée, coïncide avec la direction du rayon du cadran de cercle constitué par la partie postérieure dudit condyle. Dans ces conditions, il suffit de déplacer le canon de visée le long de ladite tige, d'une valeur égale au rayon du cercle de la partie postérieure du condyle, pour venir le positionner sur la face antérieure intra-articulaire du fémur en un point correspondant au point isométrique d'insertion. A partir de ce point, le canon de visée servira, directement ou par l'intermédiaire de douilles calibrées réglables, de guide à une broche qui sera introduite suivant la direction du même canon, de manière à guider le forage du tunnel d'insertion fémorale, par exemple au moyen d'un classique foret comportant un conduit central permettant son coulissement le long de la broche de positionnement.

Il est donc tout-à-fait évident que l'instrument ancillaire, outre ses possibilités de réglages micrométriques longitudinaux, permettant de positionner le canon de visée comme on vient de le voir, procure, dans les conditions habituelles d'intervention, au moyen d'un jeu d'inclinaisons verticales et horizontales par rapport au plan constitué par la tige d'une part, sa partie distale recourbée d'autre part, un positionnement exact aux broches et forets, pour tenir compte d'autres exigences dans la reconstruction ligamentaire ; il s'agit notamment de la direction des tunnels intra-osseux, en l'espèce des tunnels d'insertion tibiale ou fémorale, qui doit être déterminée d'une manière préférentielle, telle qu'en position d'extension du genou, le prolongement des tunnels transtibiaux et transfémoraux, soit le plus près possible de la ligne droite. Suivant des caractéristiques secondaires de l'invention, il a donc été prévu de donner des directions angulaires particulières à l'axe du canon de visée, de manière à obtenir cet alignement entre les tunnels d'insertion transtibiaux et transfémoraux, lorsque le genou est en position d'extension.

Dans une variante particulière et préférée de l'instrument ancillaire chirurgical conforme à l'invention, il est prévu d'effectuer l'intervention chirurgicale, non par une voie endo-articulaire, mais par la voie extra-articulaire, qui est préférentiellement utilisée en chirurgie arthroscopique. On sait que dans ce cas, l'intervention sera effectuée par l'extérieur du genou, ce qui évite de pratiquer des incisions profondes, et permet une reconstruction des ligaments suivant une intervention globalement bénigne.

Dans ces conditions, le repérage et le forage du tunnel d'insertion fémoral est réalisé à partir de la corticale externe du fémur, jusqu'à déboucher dans la zone d'insertion intra-articulaire, précisément au point isométrique d'insertion. Etant donné qu'il convient toujours de positionner le point isométrique d'insertion à partir du rebord postérieur du condyle fémoral externe dont on sait maintenant qu'il correspond à un cadran de cercle pratiquement parfait, l'instrument conforme à la variante de l'invention comporte une tige munie à son extrémité distale d'un crochet recourbé à angle droit, identique à la tige détaillée dans la première variante de l'instrument conforme à l'invention. L'extrémité proximale de la tige est, elle, munie d'une poignée permettant son introduction dans l'échancrure intercondylienne d'une part, et de déporter vers l'extérieur du genou, l'ensemble supportant le canon de visée, d'autre part. Cet ensemble, qui est constitué d'une barre s'étendant parallèlement à la première tige, peut coulisser au travers d'un tunnel ménagé dans l'extrémité de la poignée de ladite tige, de telle manière que le canon de visée puisse se déplacer parallèlement à ladite tige procurant la référence de l'ensemble. Naturellement, des moyens micrométriques permettent de contrôler les déplacements longitudinaux du canon de visée, et de positionner longitudinalement la sortie du même canon, de manière à l'amener au point adéquat dans la corticale externe du fémur à la sortie du tunnel d'insertion fémorale passant par le point isométrique. Le canon de visée est en outre disposé de telle manière qu'il présente une double inclinaison verticale et horizontale, pour tenir compte des exigences complémentaires déjà décrites pour la variante précédente.

En effet, l'instrument suivant cette seconde variante conforme à l'invention est utilisée de la manière suivante : le genou à opérer est présenté en position fléchie, de telle manière que le chirurgien puisse introduire la tige de référence dans l'espace intra-articulaire, jusqu'à venir disposer le crochet en position d'appui sur le rebord postérieur du condyle externe ; il est ensuite introduit, dans le tunnel de réglage à l'extrémité de la poignée, une barre coulissante supportant en bout le canon de repérage et de guidage, disposée de telle manière que le prolongement de son axe vienne couper la tige de référence en un point d'intersection tel que celui-ci soit à une distance exacte de la face d'appui du crochet égale à la valeur du rayon du rebord postérieur du condyle fémoral externe, préalablement mesuré dans des conditions déjà précisées (téléradiographie par exemple). On notera enfin que la barre coulissante supportant à son extrémité le canon de visée, présente une inclinaison verticale suffisante, pour que la direction du tunnel trans-fémoral, obtenue par forage grâce au guidage du canon de visée, soit dans l'axe de la direction du tunnel trans-tibial, lorsque le genou est en extension totale.

Il est par ailleurs à noter que quelques dispositions particulières viennent compléter la description qui vient d'être faite de cette seconde variante d'exécution, telle que par exemple, la possibilité de monter à l'intérieur du canon de visée, des douilles de divers diamètres permettant de régler avec précision la distance entre le canon de visée et la partie externe du genou ou sera pratiqué le forage du tunnel d'insertion ; ces douilles comportent intérieurement un conduit, servant de guide à la broche procurant le positionnement du foret. Les douilles peuvent être avantageusement filetées, de manière à pouvoir se visser à l'intérieur du canon de visée, et pouvoir y être déplacé axialement avec une grande précision. Naturellement des solutions équivalentes peuvent convenir en faisant, par exemple, varier la longueur de la poignée en bout de la tige de référence, de façon à régler la distance entre ladite tige et la barre coulissante du canon, pour tenir compte des différences anatomiques des genoux observées suivant chaque individu.

Enfin, il est prévu que dans les conditions d'intervention par voie extra-articulaire, la course du foret traversant depuis la partie externe jusque dans la zone d'insertion intra-articulaire, soit limitée lorsque le tunnel est totalement perforé, de manière à ne pas engendrer de blessures inutiles. Pour cela, il est disposé une concavité dans le plan vertical de la tige de référence, susceptible de constituer une butée pour l'extrémité blessante du foret, sans autoriser son ripage, pour éviter toute blessure de la masse environnante.

Il est tout à fait clair, même si cette solution n'est aujourd'hui généralement pas retenue par les praticiens, que l'on pourrait sans difficulté implanter un néo-ligament à deux faisceaux, postéro-externe et antéro-interne, pour reconstruire plus fidèlement encore le système ligamentaire biologique d'origine. Pour cela, il conviendrait encore de repérer le point isométrique de l'articulation et de doubler le canon de visée pour effectuer le forage des deux tunnels fémoraux de telle sorte qu'ils soient répartis de part et d'autre du point isométrique suivant une ligne des centres faisant environ 45° d'angle avec la verticale, pour respecter l'insertion anatomique fémorale du ligament croisé en forme de haricot centré sur ledit point isométrique.

Le deuxième but de l'invention est d'utiliser avantageusement la seconde variante de l'instrument ancillaire suivant l'invention comme guide de perçage du tunnel d'insertion tibiale dont on repère facilement, par ailleurs, l'entrée sur le plateau tibial lors de l'intervention ; en revanche, la direction longitudinale de ce tunnel trans-tibial est toujours obtenue empiriquement ; un autre objet de l'invention est donc de décrire une adaptation particulière de la variante pour effectuer correctement un seul perçage.

L'invention sera mieux comprise, et d'autres avantages ressortiront mieux encore de la description qui va suivre de deux variantes d'exécution de l'instrument ancillaire chirurgical conforme à l'invention, données ci-après à titre d'exemples non limitatifs, en référence aux dessins dans lesquels :

- la figure 1 représente une vue en coupe schématique d'une articulation du genou sur laquelle ont été mis en évidence la zone postérieure des condyles fémoraux externes, montrant leur forme circulaire et le positionnement du centre géométrique correspondant.

- la figure 2 montre une vue schématique de l'articulation du genou en position intermédiaire de flexion, mettant en évidence l'isométrie du néo-ligament en ce que la distance FT reste constante dans toutes les positions du genou.

- la figure 3 est une vue schématique de dessus du fémur reposant sur ses condyles, montrant la tige de référence de l'instrument suivant l'invention en position d'appui sur la face postérieure du condyle externe, ainsi que la position relative du point isométrique constituant l'entrée du tunnel d'insertion fémorale.

- la figure 4 est une figure schématique en élévation du condyle fémoral externe montrant le positionnement de la tige de référence de l'instrument suivant l'invention, en position d'appui sur le rebord postérieur dudit condyle, ainsi que la position relative dudit point isométrique constituant l'entrée du tunnel d'insertion transfémoral.

- la figure 5 est une vue générale d'une première variante de l'instrument ancillaire conforme à l'invention, représentée en plan muni de sa broche de positionnement.

- la figure 6 est une représentation en légère perspective de l'instrument ancillaire précédent vu préférentiellement de côté.

- la figure 7 est une vue en perspective d'une deuxième variante de l'instrument ancillaire pour le perçage du tunnel trans-fémoral montrant l'instrument en position sur le bord externe d'un condyle fémoral.

- la figure 8 est une autre vue en perspective de la variante représentée en figure 7 montrant l'instrument en position dans l'échancrure inter-condylienne.

- la figure 9 est une représentation schématique de la deuxième variante de l'instrument ancillaire des figures 7 & 8 conforme à l'invention représenté en plan.

- la figure 10 est une représentation schématique de l'instrument de la figure 9, vu de côté ; cette figure montre en outre une figure partielle et agrandie de la section transversale de la tige de référence de l'instrument, mettant en évidence sa concavité tournée vers la sortie du canon de visée, de manière à servir de butée au foret lorsqu'il débouche du tunnel d'insertion trans-fémorale.

- la figure 11 représente une adaptation de l'instrument ancillaire pour le perçage du tunnel trans-tibial suivant une direction idéale pour la fixation d'un néo-ligament.

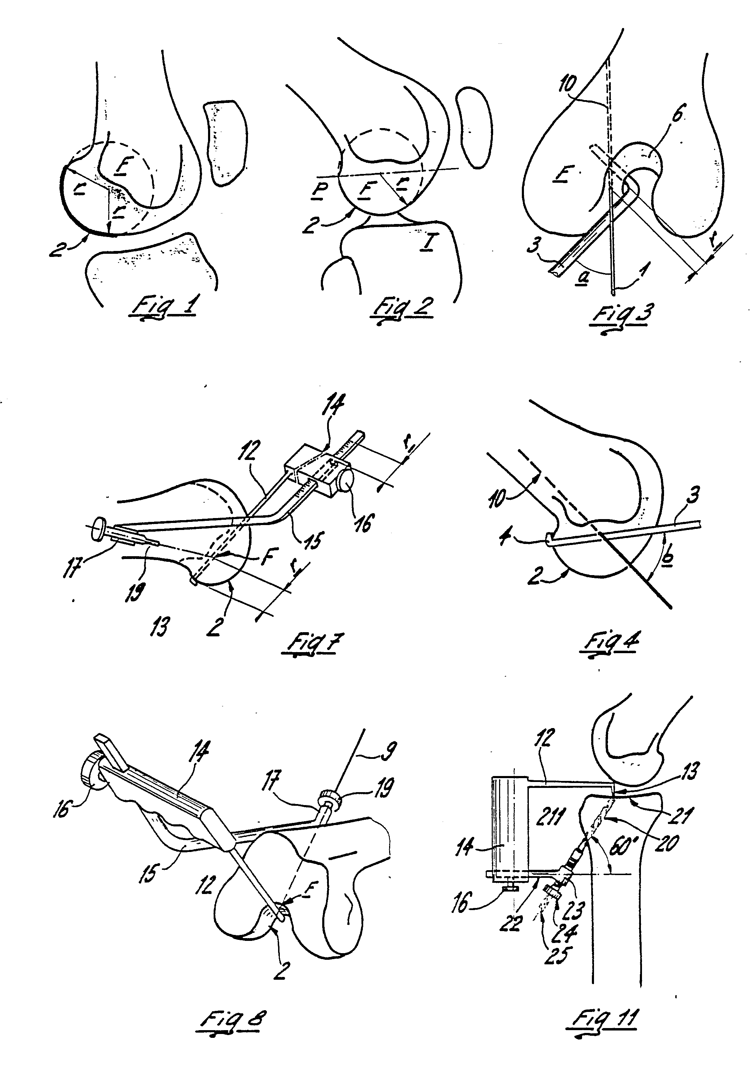

Conformément à la figure 1, on sait maintenant que la partie postérieure du condyle fémoral externe présente un profil en forme de cadran de cercle, dont on peut facilement retrouver, préalablement à l'intervention, le centre F et calculer par conséquent le rayon correspondant r ; on sait aussi que le centre F de ce cadran de cercle correspond au point isométrique pour l'insertion fémorale du néo-ligament.

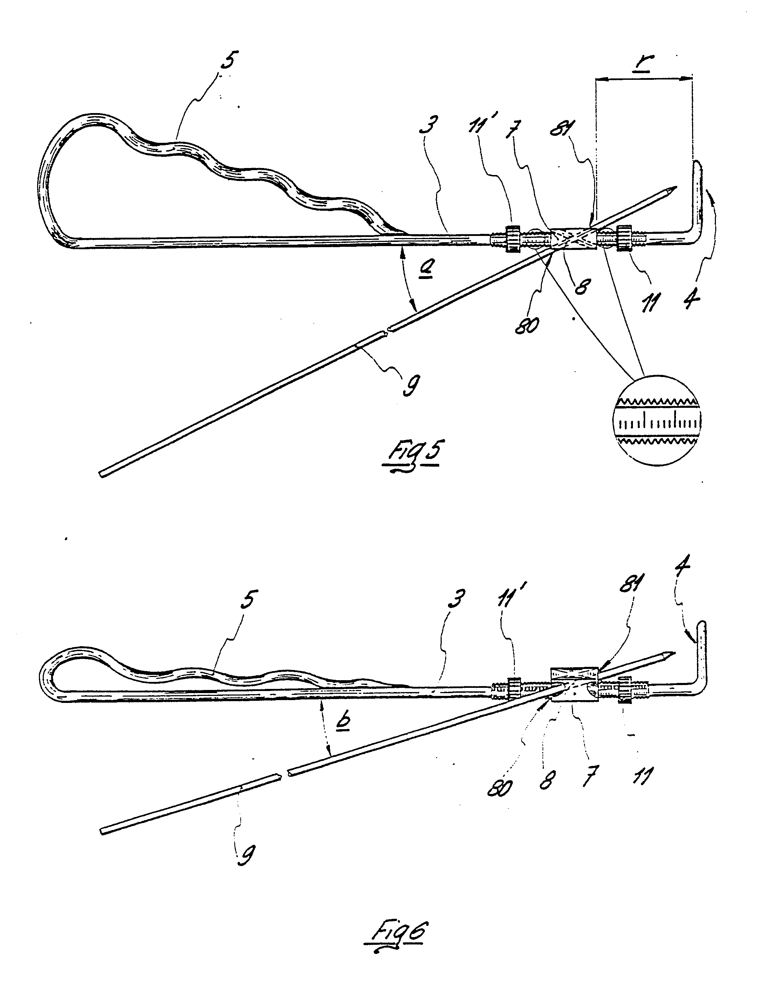

A cet égard, on a représenté sur la figure 2, la partie fémorale et tibiale de l'articulation du genou, montrant que la distance FT, comprise entre les points d'insertion fémorale et tibiale reste constante, quelle que soit la position angulaire du genou.

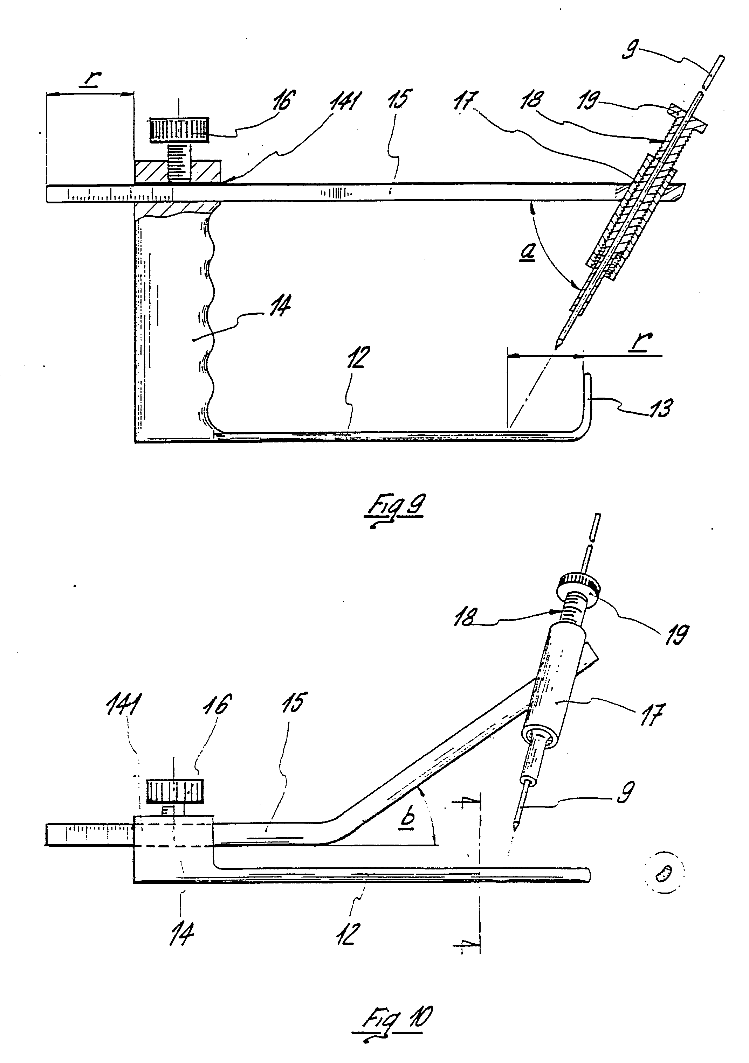

En référence aux figures 5 et 6, l'instrument ancillaire chirurgical conforme à l'invention suivant une première variante, correspondant à une intervention par voie endo-articulaire, comporte une tige 3, appelée aussi par la suite "tige de référence", présentant en son extrémité distale un crochet 4 recourbé à angle droit, sur une distance suffisante pour venir "crocheter" le rebord postérieur du condyle 1. La tige 3 comporte en son extrémité proximale une poignée 5 de forme adéquate permettant de saisir l'ensemble de l'instrument d'une part, et de guider sa pénétration dans l'échancrure intercondylienne 6, conformément à la figure 3.

Un curseur 7 peut coulisser sur la tige 3 entre deux positions écartées d'environ 3 centimètres, à partir d'une butée à droite, positionnée à environ 10 millimètres du crochet 4 venant en appui sur le rebord postérieur 2 du condyle externe 1. Le curseur 7 comporte un tunnel 8 disposé au-dessus de la tige 3 et suivant des directions angulaires verticale et horizontale définies ci-après ; en effet, le tunnel 8 est d'abord un canon de visée destiné à recevoir une broche 9 qui vient matérialiser le point isométrique F sur la face intra-articulaire du condyle 1 ; cette broche 9 sera ensuite utilisée pour déterminer la direction de forage du tunnel d'insertion trans-fémoral. Dans cette variante, la référence 8 au dessin portera indifféremment sur le canon de visée ou le tunnel qui en tient lieu.

De manière à respecter toutes les exigences requises pour une reconstruction parfaite du ligament croisé antérieur, ainsi qu'on l'a longuement expliqué en préambule, le tunnel 8 traversant le curseur 7 est tel que son axe longitudinal présente, par rapport au plan horizontal déterminé par la tige 3 et le segment 4, une inclinaison angulaire a , égale à environ 20° dans le plan horizontal compté à partir de l'axe de la tige 3, en direction de l'extrémité du segment 4, et une inclinaison b d'environ 40° dans le plan vertical, depuis un point bas correspondant à l'entrée 80 du tunnel 8, jusqu'à un point haut correspondant à la sortie 81 du même tunnel 8 en regard du segment 4.

La portion linéaire de la tige 3 sur laquelle le curseur 7 peut être librement déplacé, comporte une graduation micrométrique 9 telle que représentée dans une loupe sur la figure 5, permettant de positionner exactement la sortie 81 du tunnel 8, en regard du segment 4 à une distance égale au rayon r de la face postérieure du condyle 1 préalablement mesuré, par téléradiographie par exemple. Le curseur 7 est alors maintenu en cette position, par exemple au moyen de deux molettes 10 et 11 amenées en contact de part et d'autre du curseur 7, grâce à un filetage prévu à cet effet sur la tige 3. On notera d'ailleurs que dans une caractéristique complémentaire de l'invention, ledit filetage est avantageusement usiné au pas de 1/10ème, permettant d'utiliser chacun des filets comme repère micrométrique pour le positionnement longitudinal du curseur 7.

La partie de la tige 3 sur laquelle peut coulisser le curseur 7 présente une section carrée interdisant ainsi audit support 7 toute rotation axiale autour de la tige 3 ; toutefois, il est à noter que le curseur 7 peut être amené au-delà de la zone graduée de la tige 3 vers son extrémité proximale, de manière à y effectuer une rotation de 180°, de sorte que l'instrument peut être utilisé en prenant appui sur le rebord postérieur du condyle 1 gauche ou droite.

Suivant une seconde variante d'instrument ancillaire chirurgical suivant l'invention, permettant une reconstruction du ligament antérieur du genou par la voie extra-articulaire effectuée en principe sous arthroscopie, il est proposé une exécution particulière et d'ailleurs préférée de l'instrument, conformément aux figures 7 à 10, les figures 7 & 8 montrant l'instrument en position relative de travail par rapport aux condyles fémoraux.

L'instrument ancillaire est ici composé d'une première tige 12, comportant, à l'instar de la tige 3 dans l'exécution précédente, à son extrémité distale, un crochet 13 recourbé, suivant un angle droit ; ce crochet 13 est en tout point identique au crochet 4 de l'exécution précédente. La tige 12 comporte à son extrémité proximale une poignée 14 s'étendant perpendiculairement à l'axe de la tige 12 sur une longueur nettement supérieure à l'épaisseur d'un condyle fémoral. En bout de la poignée 14, s'étend un tunnel d'axe parallèle à celui de la tige 12, au travers duquel coulisse une barre 15 pouvant être immobilisée par une classique vis de blocage 16 ; cette barre 15 supporte en son extrémité opposée à la poignée 14, un canon 17 de visée et de guidage, disposé de telle manière que le prolongement de son axe vienne couper l'axe de la tige de référence 12.

Rappelons ici que le canon de visée et de guidage 17 doit procurer les mêmes résultats que ceux obtenus dans la première variante au moyen de la broche 9 guidée dans le tunnel 8, hormis que le forage du tunnel trans-fémoral s'effectue dans cette variante à partir de la corticale externe du fémur, la position relative dudit canon de visée et de guidage 17 par rapport aux plans définis par l'axe de la tige 12 et l'axe du segment 13, se déduit d'une première inclinaison angulaire horizontale a , égale à environ 20°, et d'une seconde inclinaison angulaire vers le haut, dans le plan vertical, correspondant à un angle de 40°, dont le sommet coïncide sensiblement avec le centre du tunnel dans la poignée de préhension 14 de la tige 12. Le réglage de l'instrument ancillaire dans cette variante d'exécution est obtenue par le coulissement de la barre 15 supportant le canon de visée 17 à l'intérieur du tunnel de guidage 18 prévu à l'extrémité de la poignée 14, ce déplacement s'effectuant suivant une direction sensiblement parallèle à l'axe de la tige de référence 12 ; de cette manière, il est possible de graduer l'extrémité proximale de la barre 15 de telle manière que l'origine de cette graduation corresponde à la position du canon de visée 17 telle que le point d'intersection entre le prolongement de son axe et l'axe de la tige de référence 12 coïncide avec l'intersection de ladite tige 12 et de la face antérieure du crochet 13 ; il apparait ainsi qu'un décalage de la barre 15 à partir de cette origine, par exemple d'une valeur r , pré-établie par les moyens que l'on a exposés auparavant, permettra d'obtenir un tunnel d'insertion trans-fémoral passant par le point isométrique F du condyle 2, dans des conditions identiques à celles obtenues à partir de l'instrument conforme à la première variante. Dans une caractéristique complémentaire, on a prévu un filetage à l'intérieur du canon de visée 17 lui permettant d'accueillir des douilles 19 de différentes dimensions pouvant être déplacées longitudinalement à l'intérieur du canon 17, par simple vissage ; en effet, on conçoit facilement que la taille des condyles fémoraux puissent varier d'un individu à l'autre, et il convient d'ajuster la longueur du canon de visée 17, pour amener son extrémité en contact avec la corticale externe du fémur. Il est évident que l'ajustement longitudinal et le maintien des douilles 19 à l'intérieur du canon de visée 17 peut être obtenu par tout autre moyen tel que, par exemple, par coulissement avec vis transversale de blocage, sans sortir du cadre de l'invention.

Il est à noter que l'on a avantageusement prévu que la section de la tige 12 soit de forme concave, tournée vers l'extérieur de l'instrument, de telle manière que ladite tige 12 puisse servir de butée finale au foret, lorsqu'il débouche du tunnel d'insertion trans-fémoral, afin d'éviter toutes blessures inutiles de la masse environnante.

On décrira maintenant une adaptation particulière de la seconde variante de l'instrument ancillaire selon l'invention permettant de l'utiliser avantageusement pour réaliser le forage du tunnel trans-tibial 20 apte à recevoir le néo-ligament à partir du plateau tibial 21 en référence au dessin de la figure 11.

L'adaptation consiste essentiellement dans le remplacement de la barre coulissante 15 équipant l'instrument ancillaire précédemment décrit par une nouvelle barre 22 supportant un canon de visée 23 permettant directement ou par l'intermédiaire d'une douille 24, le guidage d'un foret 25 destiné au forage du tunnel trans-tibial 20.

Il convient de rappeler tout d'abord que l'entrée 211 du tunnel trans-tibial 20 sur le plateau tibial 21 est relativement visible par l'opérateur lorsque le genou est en position fléchie ; la difficulté est donc de réaliser le tunnel trans-tibial 20 suivant la bonne direction de telle manière que le ligament, une fois enfoui dans les deux tunnels trans-tibiaux et trans-fémoraux, suive sensiblement une ligne droite lorsque le genou est en extension. Cette direction du canal trans-tibial, situé dans le plan médian du tibia passant par la crête antérieure de ce même tibia, est telle qu'elle fasse un angle proche de 60° par rapport au plan du plateau tibial 21.

En conséquence, on a donc prévu pour l'adaptation de l'outil ancillaire une barre 22 pouvant coulisser dans la poignée 14 de la même manière que la barre 15 dans la variante précédente avec la possibilité de blocage grâce à la vis 16 comme précédemment ; la barre 22 est droite et comporte en son extrémité libre un canon de visée 23 dont l'axe longitudinal fait un angle de 60° par rapport à l'axe de la barre 22. Le canon de visée 23 est, comme dans la variante précédente, muni intérieurement d'une douille 24 pouvant se déplacer longitudinalement au moyen par exemple d'un filetage ou de tout autre moyen de déplacement longitudinal comportant un moyen de blocage en position désirée. Dans ces conditions, lorsque la douille 24 est amenée en contact de la corticale du tibia, l'instrument étant en bonne position, comme il sera dit après, il suffit de passer à l'intérieur de la douille 24 un foret 25 pour effectuer le forage du tunnel trans-tibial 20.

Pour ce faire, l'instrument ainsi adapté, est utilisé de la manière suivante : le crochet 13 en bout de la tige 12 solidaire de la poignée 14 est disposé par sa pointe légèrement en arrière et en dedans du centre de l'insertion tibiale du ligament croisé antérieur, et la poignée 14 est elle-même tenue de telle manière qu'elle soit parallèle à la crête tibiale par l'opérateur ; compte-tenu de l'angulation du porte-foret, il ressort que dans ces conditions le tunnel qui sera réalisé aura une direction voisine de 60° par rapport au plateau tibial. Naturellement, la position de la barre 22 portant le canon de visée 23 doit être bloquée en une position telle que la direction dudit canon de visée 23 passe par l'extrémité du crochet 13 supporté par la tige 12, de manière à ce que le tunnel trans-tibial débouche bien au centre d'insertion 211 du plateau tibial 21. Il est bien évident que le forage peut être effectué soit directement avec le foret 25, soit après mise en place d'une broche, du type de la broche 9 dans les variantes précédentes, sur laquelle sera ultérieurement enfilée une mèche perforée longitudinalement.

Il est bien entendu que toutes variantes d'exécution d'ordre secondaire ne sortiraient pas du cadre de la présente invention ; l'instrument ancillaire chirurgical tel qu'il a été décrit précédemment est notamment applicable au remplacement des ligaments croisés antérieurs du genou.

**Revendications**

1 - Instrument ancillaire chirurgical pour le repérage et le forage des tunnels d'insertion fémorale et tibiale d'au moins un faisceau de néo-ligaments pour la reconstruction du ligament croisé antérieur du genou, caractérisé en ce que ledit instrument comprend une poignée (5, 14) munie à l'une de ses extrémités d'une tige rectiligne (3, 12) terminée en crochet (4, 13) perpendiculaire à ladite tige servant de référence pour au moins un canon ou tunnel de visée (8, 17, 23) prenant par rapport à ladite tige (3, 12) des directions horizontales et verticales pré-déterminées pour obtenir un alignement des directions de forage des tunnels d'insertion fémorale et tibiale lorsque le genou est en extension.

2 - Instrument ancillaire chirurgical, selon la revendication 1, d'une part pour le repérage du tunnel (10) d'insertion fémorale d'un néo-ligament (1), destiné à la reconstruction du ligament croisé antérieur du genou, l'entrée inter-condylienne dudit tunnel (10) étant située au point isométrique (F) coïncidant avant le centre du cadran de cercle correspondant au rebord postérieur (2) du condyle fémoral, et d'autre part pour le forage dudit tunnel (10) selon une direction telle qu'en position d'extension du genou, le prolongement du tunnel (10) soit sensiblement dans l'axe du tunnel d'insertion tibial, ledit instrument comprenant une tige (3, 12), comportant, à son extrémité proximale, une poignée (5, 14) et, à son extrémité distale, un crochet (4, 13) recourbé suivant un angle droit, venant au travers de l'échancrure inter-condylienne (6), en appui sur le rebord postérieur (2) du condyle fémoral concerné, pour procurer une référence de positionnement à un canon, ou tunnel, de visée et de guidage (18, 17) lié à ladite tige (3, 12), instrument caractérisé en ce que ledit canon, ou ledit tunnel, (8, 17) pré-orienté suivant des directions fixes horizontales ( a ) et verticales ( b ), peut être déplacé parallèlement à la tige (3, 12) sur une section graduée pour être immobilisé par des moyens adéquats (11, 16) de telle sorte que la distance entre la face d'appui condylienne du crochet (4, 13) et le point d'intersection entre la direction de forage et ladite tige (3, 12) soit égale au rayon ( r ) du cadran de cercle dont le centre est le point isométrique (F) déterminé préalablement, par exemple, par téléradiographie.

3 - Instrument ancillaire chirurgical suivant la revendication 2 particulièrement adapté aux reconstructions de ligament croisé antérieur du genou par voie endo-articulaire caractérisé en ce que le déplacement du canon de visée et de guidage est obtenu par le déplacement d'un curseur (7) traversé par un tunnel (8) suivant des directions horizontales ( a ) et verticales ( b ) prédéterminées, de telle façon que ledit curseur (7) puisse longitudinalement se déplacer le long de la tige (3) sur une section graduée où ledit support peut être immobilisé par des moyens adéquats (11, 11') dès lors que la distance entre la sortie (81) du tunnel (8) en regard du crochet (4) et la face antérieure d'appui sur le condyle fémoral dudit crochet (4) est égale au rayon ( r ).

4 - Instrument ancillaire chirurgical suivant la revendication 3 caractérisé en ce que, par rapport au plan horizontal déterminé par la tige (3) et le crochet (4), l'axe du tunnel (8), qui est situé au dessus de ladite tige (3), présente une inclinaison angulaire d'environ 20° dans le plan horizontal à partir de l'axe de la tige (3) vers l'extrémité du crochet (4) et une inclinaison d'environ 40° dans le plan vertical, depuis un point bas correspondant à l'entrée (80) du tunnel (8) jusqu'à un point haut correspondant à la sortie (81) du tunnel (8) qui est en regard du crochet (4).

5 - Instrument ancillaire chirurgical suivant l'une quelconque des revendications 3 ou 4, caractérisé en ce que la section de la tige (3) sur sa partie graduée est carrée, pour interdire au curseur (7) toute rotation axiale autour de la tige (3).

6 - Instrument ancillaire chirurgical suivant l'une quelconque des revendications 3 à 5, caractérisé en ce que les graduations de la tige (3) sont obtenues par les filets d'un filetage à pas millimétrique réalisé sur la section de la tige (3) sur laquelle peut se mouvoir le curseur (7), permettant aussi le déplacement longitudinal de deux vis butées (10, 11) situées devant et derrière ledit curseur (7), afin de l'immobiliser en bonne place.

7 - Instrument ancillaire chirurgical suivant la revendication 2, particulièrement adapté aux reconstructions de ligament croisé antérieur du genou par une voie extra-articulaire comportant un canon de visée et de guidage (17) déporté par des moyens adéquats et suivant des directions horizontales ( a ) et verticales ( b ) prédéterminées, à l'extérieur du genou et par conséquent de la tige (12), comportant le crochet (13) qui vient en appui sur le rebord postérieur (2) du condyle fémoral concerné (1), instrument caractérisé en ce que la direction axiale dudit canon de visée (17), depuis la corticale externe du fémur jusqu'à la zone d'insertion intercondylienne du ligament croisé antérieur, vienne couper la tige (12) en un point d'intersection situé à une distance de la face antérieure d'appui du crochet (13), égale au rayon ( r ) du cadran de cercle dont le centre est le point isométrique (F), déterminé préalablement, par exemple, par téléradiographie.

8 - Instrument ancillaire chirurgical selon la revendication 7, caractérisé en ce que la tige (12) comporte, dans sa partie proximale, une poignée (14) disposée perpendiculairement à l'axe de la tige (12), munie en son extrémité libre d'un tunnel (18) d'axe parallèle à l'axe de la tige (12) au travers duquel coulisse une barre (15) pouvant être immobilisée par une classique vis de blocage (16) qui supporte, côté du crochet (13), le canon (17), et comporte de l'autre une graduation, dont l'index de lecture est avantageusement constitué par la face arrière de la poignée (14) de la tige (12) et dont l'origine est prise telle qu'en ce point, l'intersection de la direction axiale du canon (17) avec la tige (12) coïncide avec le point d'intersection entre la tige (12) et le crochet (13).

9 - Instrument ancillaire chirurgical selon la revendication 8 caractérisé en ce la direction du canon (17) fait avec la barre coulissante (15) qui le supporte, un angle a d'environ 20°.

10 - instrument ancillaire chirurgical selon l'une quelconque des revendications 8 ou 9 caractérisé en ce que, par rapport au plan horizontal déterminé par la tige (12) et le crochet (13), la barre (15) supportant le canon (17) est décalée angulairement vers le haut, suivant un angle de 40° dont le sommet coïncide sensiblement avec le centre du tunnel (18) dans la poignée (14).

11 - instrument ancillaire chirurgical selon la revendication 1 pour le forage du tunnel trans-tibial (20) suivant une direction optimale caractérisée en ce que le canon de visée (23), situé dans le plan formé par la tige rectiligne (12) et la poignée (14) qui lui est perpendiculaire, a une direction longitudinale telle qu'elle fasse avec ladite tige (12) un angle très proche de 60° et qu'elle rencontre l'extrémité du crochet (13).

12 - Instrument ancillaire chirurgical suivant l'une quelconque des revendications 7 à 11, caractérisé en ce que le canon (17, 23) comporte un filetage intérieur, ou tout autre moyen équivalent, permettant de recevoir des douilles (19, 24) filetées, ou munies de tous moyens équivalents, dont on ajuste le déplacement dans la direction de la corticale externe du fémur, jusqu'à son contact, un canal central ménagé dans l'axe desdites douilles (19, 24) permet alors la guidage des broches (9) de forage du tunnel d'insertion.

13 - Instrument ancillaire chirurgical pour l'implantation d'un néo-ligament à deux faisceaux suivant l'une quelconque des revendications 2 à 10 ou 12, caractérisé en ce que le canon ou tunnel de visée (8, 17) est doublé de telle sorte que les deux tunnels d'insertion fémorale soient répartis de part et d'autre du point isométrique F suivant une ligne des centres faisant environ 45° d'angle avec la verticale.

EP 0 408 416 A1

Fig 1

Fig 2

Fig 3

Fig 7

Fig 4

Fig 8

Fig 11

_Fig 5_

_Fig 6_

_Fig 9_

_Fig 10_

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 292 678  (KERNFORSCHUNGSZENTRUM KARLSRUHE)<br>* Document entier *<br>--- | 1-3,7-8 ,12 | A 61 B   17/16 |
| Y | US-A-4 708 139  (W.H. DUNBAR, IV)<br>* Colonne 2, ligne 57 - colonne 3, ligne 38; colonne 4, lignes 31-55; colonne 5, lignes 19-51; colonne 6, ligne 21 - colonne 7, ligne 7; figure 1 *<br>--- | 1-3,7-8 ,12 | |
| A | EP-A-0 162 027  (M. ODENSTEN et al.)<br>* Page 3, ligne 34 - page 4, ligne 23; page 5, ligne 28 - page 6, ligne 2; figure 1 *<br>--- | 1,4,10-11,13 | |
| A | DE-C-  685 364  (O. POHRT)<br>* Page 2, colonne de gauche; figure 1 *<br>--- | 1-3,7-8 | |
| E | EP-A-0 384 098  (M. ODENSTEN et al.)<br>* Colonne 1, ligne 54 - colonne 2, ligne 29; colonne 3, lignes 27-53; colonne 4, lignes 4-38; figure 1 *<br>----- | 1,11-13 | |

**DOMAINES TECHNIQUES RECHERCHES (Int. Cl.5)**

A 61 B
A 61 F

Le présent rapport a été établi pour toutes les revendications

| Lieu de la recherche | Date d'achèvement de la recherche | Examinateur |
|---|---|---|
| LA HAYE | 05-10-1990 | NICE P.R. |